# EUROPEAN PATENT APPLICATION

(11) **EP 4 807 622 A1**
(43) Date of publication of application: **16.09.2026**
(21) Application number: 24887995.9
(22) Date of filing: 06.11.2024
(51) Int. Cl.: G06M 1/06, A61M 15/00

(54) **DOSE INDICATION DEVICE, FLUID CONTAINER AND INHALER**

(30) Priority: 07.11.2023 CN 202311475976; 07.11.2023 CN 202311475952; 07.11.2023 CN 202311468601; 07.11.2023 CN 202323007419 U
(71) Applicant: Chia Tai Tianqing Pharmaceutical Group Co., Ltd., Lianyungang, Jiangsu 222062 (CN); Chia Tai Tianqing Pharmaceutical (Guangzhou), Co., Ltd., Guangzhou, Guangdong 510700 (CN)
(72) Inventor: ZHU, Xuebing, Lianyungang, Jiangsu 222062 (CN); HE, Mingbo, Lianyungang, Jiangsu 222062 (CN); LIU, Xinhe, Lianyungang, Jiangsu 222062 (CN); GE, Yi, Lianyungang, Jiangsu 222062 (CN); ZHOU, Liping, Lianyungang, Jiangsu 222062 (CN)
(74) Representative: v. Bezold & Partner Patentanwälte - PartG mbB
(86) International application number: PCT/CN2024/130203
(87) International publication number: WO 2025/098384

(57) **Abstract**

The present application provides a dose indication device, a fluid container, and an inhaler, belonging to the field of inhaler manufacturing technologies. The dose indication device includes an outer shell and a base connected to each other; a push rod accommodating part is arranged at the center of the base, a push rod hole is provided at the center of an end surface of the push rod accommodating part, the push rod movably passes through the push rod hole, and one end of the push rod passing through the push rod hole is fixedly connected to a push-pull rod. The dose indication device further includes a transmission wheel assembly, which includes a first transmission wheel, a second transmission wheel, and a transmission shaft; the first transmission wheel is configured to be actuated to rotate in the same direction when the push-pull rod moves up and down. The dose indication device further includes a rotatable dose indicator ring which is coaxially arranged with the base, the bottom of the dose indicator ring abutting against the base, gear teeth engaged with the second transmission wheel being arranged at the top of the dose indicator ring, and graduations for displaying count values being arranged on the outer periphery of the dose indicator ring. The dose indication device of the present application can count and/or indicate the number of times the fluid container in the inhaler has been used or the remaining number of uses, and has the advantages of high structural stability, high fault tolerance rate, good versatility, low production cost, and the like.

## Description

### TECHNICAL FIELD

The present application relates to a dose indication device, a fluid container, and an inhaler, in particular to a dose indication device for a soft mist inhaler, and belongs to the field of inhaler manufacturing technologies.

### BACKGROUND

An inhaler typically includes a nebulising assembly that pumps and atomizes a fluid for inhalation by a user after a replaceable fluid container is installed in the inhaler.

After installation of the fluid container, the user may not be aware of the usage of the fluid in the fluid container. When the fluid container stores a medicinal solution for treating a chronic or acute condition, in the case where the medicinal solution in the fluid container has reached or exceeded a predetermined number of uses, if the medicinal solution is continuously used, there is a high possibility that the ejection dose is unstable and a danger may occur. Therefore, there is an urgent need for a dose indication device that can display the number of times a fluid container has been used or the remaining number of uses.

Boehringer Ingelheim (BI) has developed and launched Respimat, a propellant-free soft mist inhaler. The earliest marketed Respimat is a single-use device, and in 2019, RESPIMAT re-usable was available. This product has a reusable version, in which a replaceable container containing fluid may be inserted into an upper housing of a nebulizer and closed by a lower housing; by rotating the lower housing, a driving spring may be under tension, and the fluid can be pumped into a pressure chamber of a pressure generator. At the same time, the container moves downwards within the nebulizer. After a button is manually pressed, the driving spring is released and compresses the pressure chamber such that the fluid in the pressure chamber is at a high pressure, eventually being atomized through a nozzle while the container moves upward. A dose indication device is connected to the lower part of the container, a drive part at the bottom of the lower housing is designed to actuate the indication device when the container moves relative to the drive part, such that the indication device can be configured to count and/or indicate the number of times the container has been used or the remaining number of uses. The dose indication device involves a large number of components, and each component needs to be manufactured accurately, making the fault tolerance rate of the entire device low and the production cost high.

There are also some other types of dose indicators in the prior art that are designed to achieve a dose indicating function by means of electronics, the use of which increases cost and may have battery life issues. In addition, these dose indicator structures are relatively complex, require a large number of small mechanical components, are costly, and are difficult to assemble.

In addition, although the dose indicator may allow the user to observe the use of the inhaler, the user may sometimes forget to observe, and if the fluid in the fluid container is nearly depleted or has been depleted, the discharged dose of the inhaler is different from the predetermined dose, which makes it dangerous for the user to continue using the inhaler.

In addition, in order to improve convenience and reduce the number of times the user replaces the fluid container, each fluid container usually contains as much administered dose as possible, and the design space of the dose indicator is limited, which further increases the precision requirements for the components in the device and increases the production difficulty.

Therefore, there is also an urgent need for a dose indication device that has a simple structure, a high fault tolerance rate and good versatility, can display the number of uses or the remaining number of uses of the fluid container, and locks the fluid container after the fluid container reaches a predetermined number of uses, especially suitable for fluid containers containing a large amount of administered dose.

### SUMMARY

In view of the shortcomings of the prior art, the present application provides the following solutions:
In a first aspect, the present application provides a dose indication device for an inhaler. The inhaler includes a housing for receiving a fluid container and a nebulising assembly for nebulising a fluid, a drive part for actuating a dose indication device being arranged at a bottom inside the housing. The dose indication device includes an outer shell and a base connected to each other, and an empty cavity located between the outer shell and the base; the dose indication device is connected to the fluid container through the outer shell, a push rod accommodating part protruding toward the outer shell is arranged at a center of the base, a push rod hole penetrating the base and an end surface of the push rod accommodating part away from the base is provided at a center of the end surface, a push rod movably passes through the push rod hole, and a push-pull rod is fixedly connected to an end of the push rod passing through the push rod hole and facing the outer shell; the push-pull rod includes a push-pull rod main body, and a pushing rod and a pulling rod which are located on two sides of the push-pull rod main body and arranged opposite each other, a height of the pushing rod being lower than a height of the pulling rod. The dose indication device further includes a transmission wheel assembly, and the transmission wheel assembly includes a first transmission wheel, a second transmission wheel, and a horizontally arranged transmission shaft; the first transmission wheel is fixed in a middle of the transmission shaft and located between the pushing rod and the pulling rod, a distance between the pushing rod and the pulling rod is smaller than an outer diameter of the first transmission wheel, and the first transmission wheel is configured to be actuated by the pushing rod or the pulling rod to rotate in a same direction when the push-pull rod moves up and down; the second transmission wheel is fixed at an end portion of the transmission shaft. The dose indication device further includes a dose indicator ring coaxially arranged with the base and rotatable around a central axis of the base, a bottom of the dose indicator ring abutting against the base, gear teeth engaged with the second transmission wheel being arranged at a top of the dose indicator ring, and graduations for displaying count values being arranged on an outer periphery of the dose indicator ring.

Preferably, a length of the push rod does not exceed a length of the push rod hole.

Preferably, the push rod does not protrude from a surface of the base away from the outer shell.

To move and reset the push rod, a stop part is arranged at the push rod, an elastic assembly is further arranged between the push rod and the end surface, one end of the elastic assembly abuts against the stop part, and the other end thereof abuts against the end surface. In order to provide a sufficient distance for the push rod to move, preferably, the stop part is located at the bottom of the push rod.

In order to push the first transmission wheel to rotate, the pushing rod includes a pushing part, the pushing part being fixedly connected to the push-pull rod main body by a first vertical rod arranged vertically; the pulling rod includes a pulling part, the pulling part being fixedly connected to the push-pull rod main body by a second vertical rod arranged vertically; a height of the pushing part is lower than a height of the pulling part. Preferably, the pushing part is fixedly connected to the push-pull rod main body through two first vertical rods, and the two first vertical rods are connected to two ends of the pushing part, respectively; the pulling part is fixedly connected to the push-pull rod main body through two second vertical rods, and the two second vertical rods are connected to two ends of the pulling part, respectively.

In order to ensure that the transmission wheel assembly can be effectively actuated and improve the fault tolerance rate, preferably, a ratio of the distance between the pushing rod and the pulling rod to a radius of the first transmission wheel ranges from 1.3 to 1.9, further preferably from 1.4 to 1.6, and even further preferably from 1.4 to 1.5; and/or
a ratio of a height difference between the pushing rod and the pulling rod to a radius of the first transmission wheel is greater than or equal to 0.4, or greater than or equal to 0.5, or greater than or equal to 0.6, or greater than or equal to 0.7, or greater than or equal to 0.8, or greater than or equal to 0.9, or greater than or equal to 1, and further preferably ranges from 0.6 to 0.8; and/or
a ratio of the height of the pushing rod to a radius of the first transmission wheel ranges from 0.6 to 1.3, further preferably from 0.8 to 1.2, and even further preferably from 1 to 1.1.

In order to provide a more stable acting force and improve the fault tolerance rate, preferably, teeth of the first transmission wheel are deflected towards the pushing rod, with a deflection angle α of 0° to 70°, further preferably 15° to 65°, still further preferably 30° to 65°, and even further preferably 45° to 55°.

Preferably, the pushing part and the pulling part are arranged horizontally, or the pushing part and the pulling part are arranged obliquely in a horizontal direction towards the pushing rod, with an inclination angle β of 0° to 60°, further preferably 0° to 45°, still further preferably 10° to 30°, and even further preferably 15° to 25°.

Preferably, the dose indication device includes a transmission wheel assembly bracket fixed to the base, the transmission wheel assembly being rotatably arranged in the transmission wheel assembly bracket.

On the basis of different situations, the number of the teeth of the first transmission wheel is 3 to 6, and the number of teeth of the second transmission wheel is less than the number of the teeth of the first transmission wheel. Preferably, the number of the teeth of the second transmission wheel is 2 to 5, and further preferably 2.

In order to prevent the first transmission wheel from rotating reversely such that the dose indication device displays an error, the dose indication device includes lock pawls for preventing reverse rotation of the first transmission wheel, the lock pawls being arranged on a top surface of the outer shell and extending towards the first transmission wheel. Preferably, the dose indication device includes two lock pawls extending towards two sides of a tooth of the first transmission wheel, respectively.

In order to define the position of the dose indicator ring, the base is provided with a plurality of limiting sheets, and the plurality of limiting sheets are arranged on the outer periphery of the dose indicator ring; an observation window is arranged on a side surface of the outer shell, and the position of the observation window is arranged corresponding to the position of the dose indicator ring.

In order to prevent the dose indicator ring from rotating reversely such that the dose indication device displays an error, a ratchet mechanism for preventing reverse rotation of the dose indicator ring is arranged on the inner periphery of the dose indicator ring, and the ratchet mechanism includes a ratchet and check pawls.

In order to prevent a user from continuing to use the fluid container when the fluid is nearly depleted or has been depleted, the dose indication device includes a locking mechanism, the locking mechanism being configured to prevent movement of the push rod and/or rotation of the dose indicator ring after the fluid container has been used a predetermined number of times, thereby locking the fluid container and prompting the user to replace the fluid container with a new one.

In summary, in the first aspect, the present application provides a dose indication device. By means of components such as a push rod, a push-pull rod, a transmission wheel assembly, and a dose indicator ring that is provided with gear teeth, when a user uses an inhaler, an up-down motion of a fluid container in the perpendicular direction is converted into a rotational motion of the transmission wheel assembly in the perpendicular direction by using the push rod and the push-pull rod, and then into a rotational motion of the dose indicator ring in the horizontal direction. For different products or different batches of the same product, even if the initial position of the assembled fluid container relative to the drive part is different and/or the relative movement distance is different, the present application can convert a longitudinal motion within a certain range into a rotational motion of the gear teeth by a specific angle. Compared with the traditional products that require precise control of the sizes of components, the present application increases the fault tolerance rate of the dose indication device, improves the versatility, and reduces the production cost, and moreover, the structure of the entire dose indication device is simple and the stability is high. In the present application, a locking mechanism is further provided, which can effectively and firmly lock the fluid container after a predetermined number of uses, thereby preventing the user from continuing to use the fluid container when the fluid is nearly depleted or has been depleted, improving the safety, and also reminding the user to replace the fluid container with a new one.

In a second aspect, the present application provides a dose indication device for an inhaler. The inhaler includes a housing for receiving a fluid container and a nebulising assembly for nebulising a fluid, a drive part for actuating a dose indication device being arranged at a bottom inside the housing. The dose indication device includes an outer shell and a base connected to each other, and an empty cavity located between the outer shell and the base; the dose indication device is connected to the fluid container through the outer shell, a push rod accommodating part protruding toward the outer shell is arranged at a center of the base, a push rod hole penetrating the base and an end surface of the push rod accommodating part away from the base is provided at a center of the end surface, and a push rod movably passes through the push rod hole. The dose indication device further includes a dose indicator ring coaxially arranged with the base and rotatable around a central axis of the base, graduations for displaying count values are arranged on an outer periphery of the dose indicator ring, and the push rod drives the dose indicator ring to rotate through a transmission mechanism. The dose indication device further includes a locking mechanism, and the locking mechanism includes a rotatable stopper, the stopper being configured to prevent movement of the push rod and/or rotation of the dose indicator ring after the fluid container has been used a predetermined number of times.

Preferably, an observation window corresponding to the dose indicator ring is arranged on a side surface of the outer shell.

To move and reset the push rod, a stop part is arranged at the push rod, an elastic assembly is further arranged between the push rod and the end surface, one end of the elastic assembly abuts against the stop part, and the other end thereof abuts against the end surface. In order to provide a sufficient distance for the push rod to move, preferably, the stop part is located at the bottom of the push rod.

Preferably, the locking mechanism includes a rotatable stopper, a vertically arranged stopper rotating shaft is arranged on the base, the stopper is rotatably sleeved on the stopper rotating shaft, and the stopper includes an abutment part, a limiting part, and a pushing abutment part, the abutment part and the limiting part being located on two sides of the stopper rotating shaft, and the pushing abutment part being arranged close to the dose indicator ring.

In order to push the stopper to rotate, the locking mechanism includes a pushing member arranged on an inner periphery of the dose indicator ring, the pushing member is configured to push the stopper from a preset position to an abutment position after the dose indicator ring rotates by a preset angle, the stopper, when in the preset position, does not prevent the movement of the push rod, and the stopper, when in the abutment position, prevents the movement of the push rod.

Preferably, the pushing member is a protrusion protruding from the inner periphery of the dose indicator ring towards the stopper, the protrusion is arranged corresponding to the pushing abutment part, and the protrusion is configured to push the pushing abutment part after the dose indicator ring rotates by a preset angle.

Preferably, a stopper opening hole communicating with the push rod hole is provided on a side surface of the push rod accommodating part, the stopper opening hole is provided corresponding to the abutment part such that the abutment part can pass through the stopper opening hole when the stopper rotates, the abutment part, when in the preset position, does not pass through the stopper opening hole, and the abutment part, when in the abutment position, passes through the stopper opening hole and at least partially protrudes from the push rod hole.

Preferably, in the abutment position, the bottom of the push rod is located above the stopper opening hole.

Preferably, in the abutment position, the bottom of the push rod is located below the stopper opening hole.

In order to prevent the stopper from rotating unnecessarily, a limiting mechanism is arranged on the base, the limiting mechanism is arranged corresponding to the limiting part, and the limiting mechanism is configured to retain the stopper in the preset position.

Preferably, the limiting part is a semicircular boss, the limiting mechanism is a limiting slot provided on the base, and the boss and the limiting slot are correspondingly provided in a matching shape.

Alternatively, the limiting mechanism is an elastic sheet arranged on a surface of the base facing the stopper and extending obliquely toward the stopper, and a portion of the stopper facing the elastic sheet is used as the limiting part.

The base is provided with a protruding point for preventing the abutment part from moving away from the stopper opening hole, and the protruding point abuts against the abutment part when the stopper is in the preset position.

Preferably, the dose indication device further includes a stopping member, and the stopping member, when in the abutment position, prevents further rotation of the stopper.

Further preferably, the stopping member is a convex point arranged on the base.

Further preferably, a hole wall on one side of the stopper opening hole may serve as a stopping member.

Further preferably, the limiting slot may serve as a stopping member.

Exemplarily, the transmission mechanism includes:
a push-pull rod, the push-pull rod being fixedly connected to an end of the push rod passing through the push rod hole and facing the outer shell; the push-pull rod including a push-pull rod main body, and a pushing rod and a pulling rod which are located on two sides of the push-pull rod main body and arranged opposite each other, a height of the pushing rod being lower than a height of the pulling rod; and
a transmission wheel assembly, including a first transmission wheel, a second transmission wheel, and a horizontally arranged transmission shaft; the first transmission wheel being fixed in a middle of the transmission shaft and located between the pushing rod and the pulling rod, a distance between the pushing rod and the pulling rod being smaller than an outer diameter of the first transmission wheel, and the first transmission wheel being configured to be actuated by the pushing rod or the pulling rod to rotate in a same direction when the push-pull rod moves up and down; the second transmission wheel being fixed at an end portion of the transmission shaft;
where a bottom of the dose indicator ring abuts against the base, and gear teeth engaged with the second transmission wheel are arranged at a top of the dose indicator ring.

In summary, in the second aspect, the present application further provides a dose indication device. A locking mechanism is provided, which can effectively and firmly lock the fluid container after a predetermined number of uses, thereby preventing the user from continuing to use the fluid container when the fluid is nearly depleted or has been depleted, improving the safety, and also reminding the user to replace the fluid container with a new one.

The locking mechanism in the present application provides a double locking effect, in one aspect, the locking mechanism locks the count of the dose indication device by the stopper preventing the up and down movement of the push rod, and in another aspect, the locking mechanism prevents further rotation of the stopper by the stopping member, such that the pushing abutment part provides a reverse blocking effect on the pushing member, thereby preventing the rotation of the dose indicator ring and thus locking the indication of the dose indication device.

In a third aspect, the present application provides a dose indication device, which includes: an outer shell and a base connected to each other, and an empty cavity located between the outer shell and the base; a transmission wheel assembly, which includes a first transmission wheel, a second transmission wheel, and a horizontally arranged transmission shaft, where the first transmission wheel is fixed in a middle of the transmission shaft and arranged corresponding to the push-pull rod, and the first transmission wheel is configured to be actuated to rotate in the same direction when counting is required; and the second transmission wheel is fixed at an end portion of the transmission shaft; and a dose indicator ring, which is coaxially arranged with the base and rotatable around a central axis of the base, where a bottom of the dose indicator ring abuts against the base, gear teeth engaged with the second transmission wheel are arranged at a top of the dose indicator ring, and graduations for displaying count values are arranged on an outer periphery of the dose indicator ring.

Further, the number of the teeth of the second transmission wheel is greater than the number of the teeth of the first transmission wheel. Preferably, the number of the teeth of the second transmission wheel is twice the number of the teeth of the first transmission wheel. This design can significantly improve the display accuracy of the device when there are fewer graduations for displaying count values on the outer periphery of the dose indicator ring, i.e., there are fewer numerical values to be counted.

Further, the number of the teeth of the second transmission wheel is less than or equal to the number of the teeth of the first transmission wheel.

To improve the fault tolerance rate and ensure the stability of the structure of the transmission wheel assembly, preferably, the number of the teeth of the second transmission wheel is less than the number of the teeth of the first transmission wheel. This design is more advantageous when there are more graduations for displaying count values on the outer periphery of the dose indicator ring, i.e., there are more numerical values to be counted.

Further preferably, the number of the teeth of the first transmission wheel is 3 to 6.

Further preferably, the number of the teeth of the second transmission wheel is 2 to 5.

Even further preferably, the number of the teeth of the second transmission wheel is 2, with an included angle between the two teeth of 144°.

Preferably, the dose indication device includes a transmission wheel assembly bracket fixed to the base, the transmission wheel assembly being rotatably arranged in the transmission wheel assembly bracket.

Further, the dose indication device further includes a push-pull rod that can move up and down in the empty cavity.

Still further, the first transmission wheel is configured to be actuated to rotate in the same direction when the push-pull rod moves up and down.

To more effectively push the first transmission wheel to rotate, the push-pull rod includes a push-pull rod main body, and a pushing rod and a pulling rod which are located on two sides of the push-pull rod main body and are arranged opposite each other. The height of the pushing rod is less than the height of the pulling rod, and the first transmission wheel is located between the pushing rod and the pulling rod. Preferably, the pushing part is fixedly connected to the push-pull rod main body through two first vertical rods, and the two first vertical rods are connected to two ends of the pushing part, respectively; the pulling part is fixedly connected to the push-pull rod main body through two second vertical rods, and the two second vertical rods are connected to two ends of the pulling part, respectively.

In order to provide a more stable acting force and improve the fault tolerance rate, preferably, teeth of the first transmission wheel are deflected towards the pushing rod, with a deflection angle α of 0° to 70°, further preferably 15° to 65°, still further preferably 30° to 65°, and even further preferably 45° to 55°.

In order to ensure that the transmission wheel assembly can be effectively actuated and improve the fault tolerance rate, preferably, a ratio of the distance between the pushing rod and the pulling rod to a radius of the first transmission wheel ranges from 1.3 to 1.9; and/or
a ratio of a height difference between the pushing rod and the pulling rod to a radius of the first transmission wheel ranges from 0.6 to 0.8; and/or
a ratio of the height of the pushing rod to a radius of the first transmission wheel ranges from 0.6 to 1.3.

Preferably, the pushing part and the pulling part are arranged horizontally, or the pushing part and the pulling part are arranged obliquely in a horizontal direction towards the pushing rod, with an inclination angle β of 0° to 60°, further preferably 0° to 45°, still further preferably 10° to 30°, and even further preferably 15° to 25°.

Preferably, a push rod accommodating part protruding toward the outer shell is arranged at a center of the base, a push rod hole penetrating the base and an end surface of the push rod accommodating part away from the base is provided at a center of the end surface, a push rod movably passes through the push rod hole, and the push-pull rod is fixedly connected to an end of the push rod passing through the push rod hole and facing the outer shell.

Preferably, a length of the push rod does not exceed a length of the push rod hole.

Preferably, the push rod does not protrude from a surface of the base away from the outer shell.

In order to prevent the first transmission wheel from rotating reversely such that the dose indication device displays an error, the dose indication device includes lock pawls for preventing reverse rotation of the first transmission wheel, the lock pawls being arranged on a top surface of the outer shell and extending towards the first transmission wheel. Preferably, the dose indication device includes two lock pawls extending towards two sides of a tooth of the first transmission wheel, respectively.

In order to prevent the dose indicator ring from rotating reversely such that the dose indication device displays an error, a ratchet mechanism for preventing reverse rotation of the dose indicator ring is arranged on the inner periphery of the dose indicator ring, and the ratchet mechanism includes a ratchet and check pawls.

In order to define the position of the dose indicator ring, the base is provided with a plurality of limiting sheets, and the plurality of limiting sheets are arranged on the outer periphery of the dose indicator ring; an observation window is arranged on a side surface of the outer shell, and the position of the observation window is arranged corresponding to the position of the dose indicator ring.

In order to prevent a user from continuing to use the fluid container when the fluid is nearly depleted or has been depleted, the dose indication device includes a locking mechanism, the locking mechanism being configured to prevent movement of the push rod and/or rotation of the dose indicator ring after the fluid container has been used a predetermined number of times, thereby locking the fluid container and prompting the user to replace the fluid container with a new one.

In summary, in the third aspect, the present application further provides a dose indication device. By means of the matching design of the first transmission wheel, the second transmission wheel, and the dose indicator ring, in one aspect, the force of the push-pull rod can be effectively transmitted to the dose indicator ring, the structure is simple, and the stability is high; in another aspect, by means of the design of the teeth on the first transmission wheel and the second transmission wheel, and the design of the gear teeth on the dose indicator ring, firstly, the deflection design of the teeth on the first transmission wheel improves the fault tolerance rate between the push-pull rod and the first transmission wheel; secondly, by using the difference in the number of the teeth on the first transmission wheel and the second transmission wheel, the transmission ratio of the device can be adjusted on the basis of the number of counted values, such that the flexibility of the entire device is improved, which is especially advantageous for fluid containers containing a large amount of administered dose, and in addition, the number of corresponding gear teeth on the dose indicator ring can be significantly reduced, such that the structural strength of the gear teeth can be enhanced in a limited space, the structural stability of the device is improved, and the production difficulty and production cost are reduced; thirdly, the rotation angle corresponding to an individual gear tooth on the dose indicator ring can be increased, and the meshing space of the gear teeth and the teeth on the second transmission wheel can be increased, thereby further improving the fault tolerance rate of the device.

In a fourth aspect, the present application provides a dose indication device, which includes: an outer shell and a base connected to each other, and an empty cavity located between the outer shell and the base; a transmission wheel assembly, which includes a first transmission wheel, the first transmission wheel being configured to be actuated to rotate in the same direction when counting is required; a dose indicator ring, which is coaxially arranged with the base and is configured to be actuated to rotate around a central axis of the base when the first transmission wheel rotates in the same direction, graduations for displaying count values being arranged on an outer periphery of the dose indicator ring; a first anti-reverse-rotation mechanism, which is arranged in the empty cavity and extends towards the first transmission wheel to prevent reverse rotation of the first transmission wheel; and a second anti-reverse-rotation mechanism, which is arranged on an inner side of the dose indicator ring to prevent reverse rotation of the dose indicator ring.

Further, the first anti-reverse-rotation mechanism includes at least one lock pawl.

Still further, the lock pawls are arranged in the empty cavity and extend toward the first transmission wheel.

To facilitate design and provide compactness of a device structure, further, the lock pawls are arranged on a top surface of the outer shell and extend toward the first transmission wheel.

To improve the structural stability, preferably, the first anti-reverse-rotation mechanism includes two lock pawls, and the two lock pawls extend towards two sides of a tooth of the first transmission wheel, respectively.

Further, the lock pawl is of an elastic sheet structure. When the first transmission wheel rotates in the same direction, the lock pawls are slightly elastically deformed and provide an audible feedback. When the first transmission wheel rotates in a reverse direction, the lock pawls are greatly deformed and generate a large reaction force, thereby preventing reverse rotation of the first transmission wheel.

Preferably, the lock pawls extend towards the tooth of the first transmission wheel and have a minimum thickness at a position close to the tooth of the first transmission wheel.

In order to facilitate the anti-reverse-rotation action of the lock pawls on the first transmission wheel, preferably, the teeth of the first transmission wheel are deflected in an opposite direction of a rotation direction of the first transmission wheel, with a deflection angle α of 0° to 70°, further preferably 15° to 65°, still further preferably 30° to 65°, and even further preferably 45° to 55°.

In order to prevent the dose indicator ring from rotating reversely such that the dose indication device displays an error, further, the second anti-reverse-rotation mechanism is a ratchet mechanism. Further, a ratchet is arranged on an inner periphery of the dose indicator ring, and mating check pawls are arranged in the empty cavity.

Preferably, the number of the check pawls is at least two; further preferably, the number of the check pawls is two, and the two check pawls are arranged symmetrically with respect to each other. Further, the transmission wheel assembly further includes a second transmission wheel and a horizontally arranged transmission shaft; the first transmission wheel is fixed in a middle of the transmission shaft, and the second transmission wheel is fixed at an end portion of the transmission shaft. Further, gear teeth engaged with the second transmission wheel are arranged at a top of the dose indicator ring.

Preferably, the dose indication device includes a transmission wheel assembly bracket fixed to the base, the transmission wheel assembly being rotatably arranged in the transmission wheel assembly bracket.

In order to define the position of the dose indicator ring, the base is provided with a plurality of limiting sheets, and the plurality of limiting sheets are arranged on the outer periphery of the dose indicator ring; an observation window is arranged on a side surface of the outer shell, and the position of the observation window is arranged corresponding to the position of the dose indicator ring.

In summary, in the fourth aspect, the present application provides a dose indication device for an inhaler. The dose indication device has a dual anti-reverse-rotation design, effectively ensuring the accuracy of counting and/or displaying. In one aspect, reverse rotation of the first transmission wheel is prevented by the lock pawls, improving the counting accuracy of the device, and in another aspect, reverse rotation of the dose indicator ring is prevented by the ratchet mechanism, improving the display accuracy of the device.

In a fifth aspect, the present application provides a fluid container. The fluid container is connected to the dose indication device as described in the first to fourth aspects of the present application.

Further, the dose indication device is connected to the fluid container through the outer shell, and preferably, the dose indication device is connected to the bottom of the fluid container through the outer shell.

Preferably, the fluid container is fixedly connected to the dose indication device, and when the fluid container needs to be replaced with a new one, the fluid container and the dose indication device are replaced together.

In a sixth aspect, the present application provides an inhaler, a housing of the inhaler being provided with the dose indication device as described in the first to fourth aspects of the present application.

Further, the housing of the inhaler may receive a fluid container to which the dose indication device described above is connected.

In a seventh aspect, the present application further provides a method for counting and/or indicating the number of times the fluid container in the inhaler has been used or the remaining number of uses by using the dose indication device described above.

In an eighth aspect, the present application further provides a dose indication device having the structure as described in the first to fourth aspects of the present application. It can be understood that the dose indication device can be used in other devices or equipment requiring counting and/or displaying, apart from an inhaler, as long as the device or equipment is provided with a drive part capable of actuating the dose indication device. Similarly, the dose indication device may be connected to the fluid container through the outer shell, or may also be connected to other components in the device or equipment through the outer shell, or even the dose indication device may be present individually in the device or equipment, as long as the drive part can actuate the dose indication device during motions requiring counting and/or displaying.

The technical solutions of the present application will be described in detail below with reference to the accompanying drawings and specific embodiments.

### BRIEF DESCRIPTION OF THE DRAWINGS

FIG. 1 is a schematic structural diagram of an inhaler according to the present application.
FIG. 2 is a cutaway view of the inhaler according to the present application.
FIG. 3 is a schematic exploded view of a dose indication device for the inhaler according to the present application.
FIG. 4 is a cross-sectional view illustrating an internal structure of the dose indication device for the inhaler.
FIG. 5 is a schematic cross-sectional view of the dose indication device in a ready-to-dose state.
FIG. 6 is a schematic cross-sectional view of the dose indication device in a priming dose state.
FIG. 7 is a schematic structural diagram of a transmission wheel assembly.
FIG. 8 is a schematic cross-sectional view of a first transmission wheel.
FIG. 9 is a schematic diagram illustrating the engagement of a second transmission wheel and a dose indicator ring.
FIG. 10 is a schematic diagram illustrating the cooperation between an observation window and a dose indicator ring.
FIG. 11 is a top view illustrating a ratchet mechanism.
FIG. 12 is a schematic diagram illustrating a stopper in a preset position, illustrating an example of a locking mechanism.
FIG. 13 is a schematic diagram illustrating the stopper in an abutment position, illustrating an example of the locking mechanism.
FIG. 14 is a schematic diagram illustrating the stopper in the preset position, illustrating another example of the locking mechanism.
FIG. 15 is a schematic diagram illustrating the stopper in the abutment position, illustrating another example of the locking mechanism.
FIG. 16 is a schematic structural diagram illustrating an elastic structure, illustrating another example of the locking mechanism.

### DETAILED DESCRIPTION

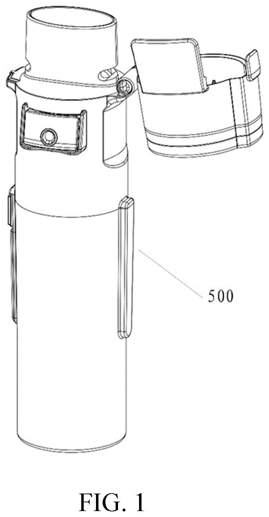

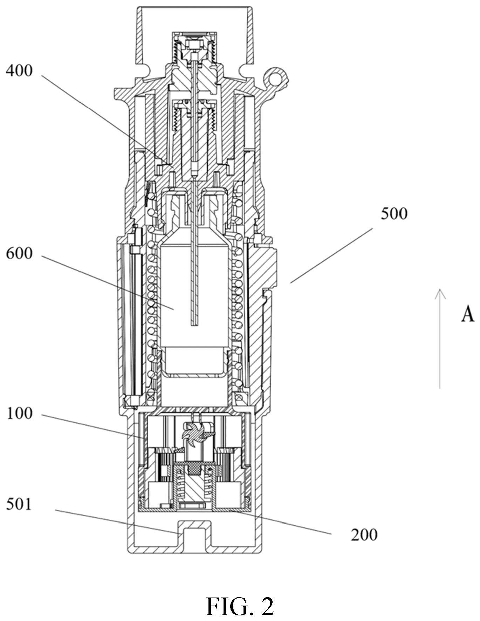

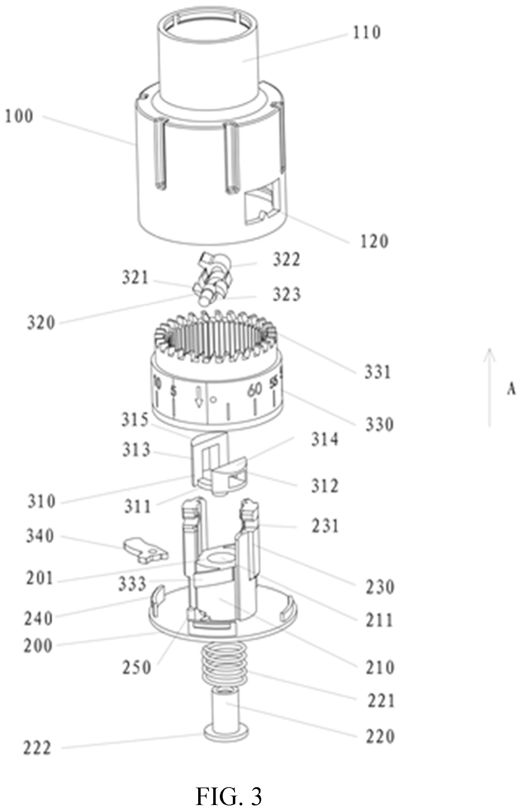

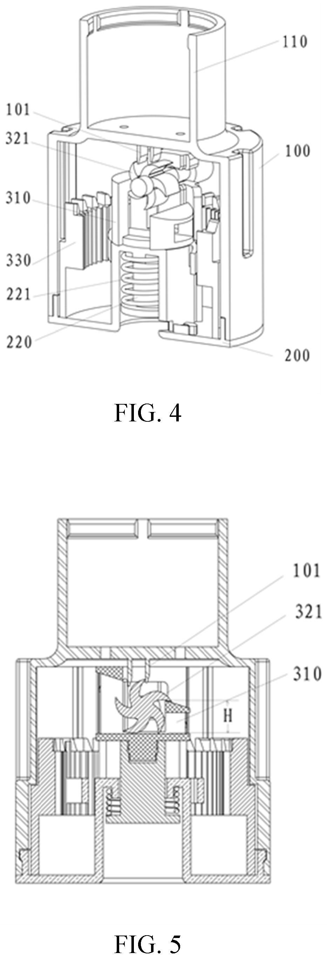

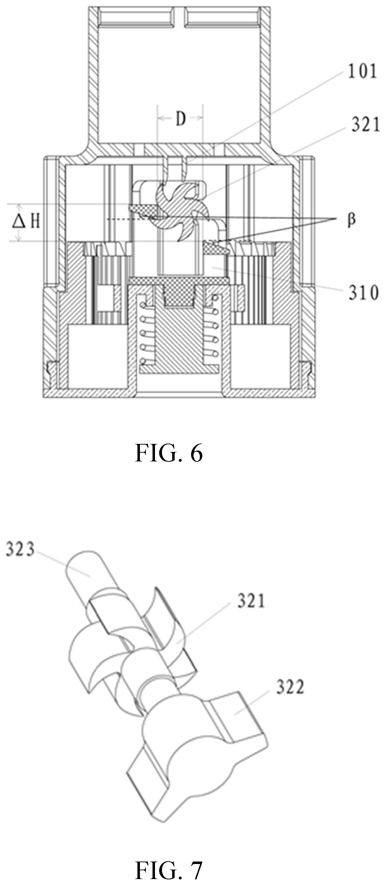

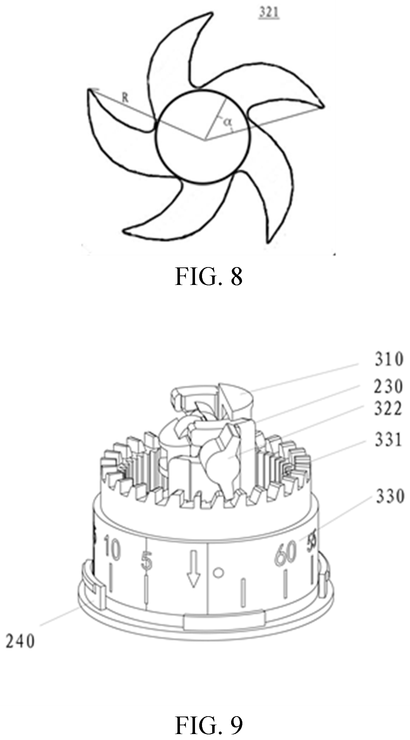

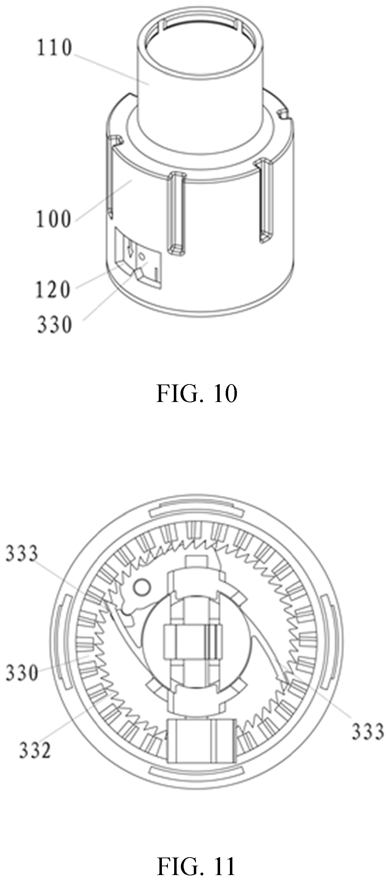

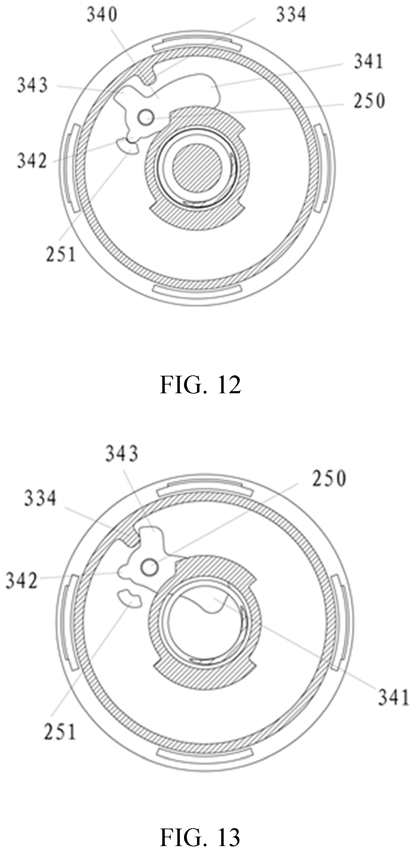

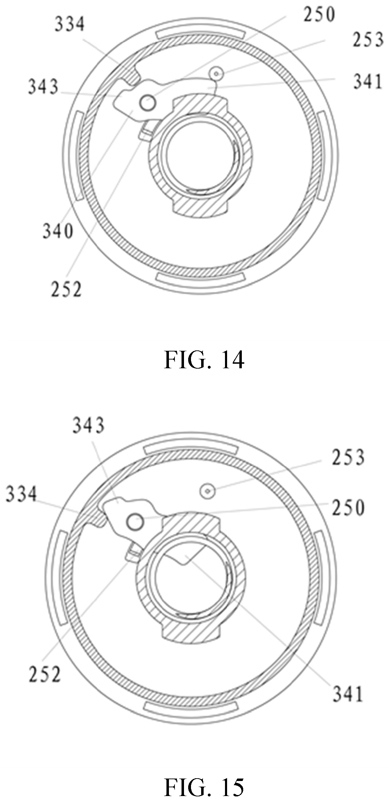

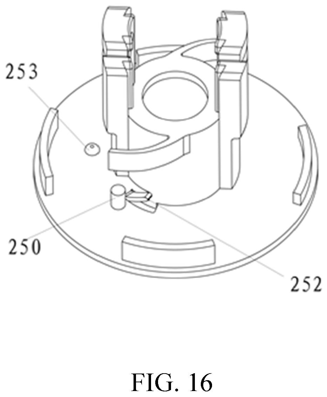

FIG. 1 is a schematic structural diagram of an inhaler according to the present application; FIG. 2 is a cutaway view of the inhaler according to the present application; FIG. 3 is a schematic exploded view of a dose indication device for the inhaler according to the present application; FIG. 4 is a cross-sectional view illustrating an internal structure of the dose indication device for the inhaler; FIG. 5 is a schematic cross-sectional view of the dose indication device in a ready-to-dose state; FIG. 6 is a schematic cross-sectional view of the dose indication device in a priming dose state; FIG. 7 is a schematic structural diagram of a transmission wheel assembly; FIG. 8 is a schematic cross-sectional view of a first transmission wheel; FIG. 9 is a schematic diagram illustrating the engagement of a second transmission wheel and a dose indicator ring; FIG. 10 is a schematic diagram illustrating the cooperation between an observation window and a dose indicator ring; FIG. 11 is a top view illustrating a ratchet mechanism; FIG. 12 is a schematic diagram illustrating a stopper in a preset position, illustrating an example of a locking mechanism; FIG. 13 is a schematic diagram illustrating the stopper in an abutment position, illustrating an example of the locking mechanism; FIG. 14 is a schematic diagram illustrating the stopper in the preset position, illustrating another example of the locking mechanism; FIG. 15 is a schematic diagram illustrating the stopper in the abutment position, illustrating another example of the locking mechanism; and FIG. 16 is a schematic structural diagram illustrating an elastic structure, illustrating another example of the locking mechanism. As shown in FIGs. 1-16, the present application provides a dose indication device for an inhaler. The dose indication device is applicable to a reusable inhaler, and includes, but is not limited to, RESPIMAT^{®} re-usable by Boehringer Ingelheim (BI) or an inhalation device disclosed in Patent Application No. 201880027066.6.

Exemplarily, as shown in FIGs. 1 and 2, the inhaler 500 includes a housing for receiving a fluid container 600 (such as a medicine bottle) and a nebulising assembly 400 for nebulising a fluid, a drive part 501 (e.g., a small rod, a protrusion, etc.) being arranged at the bottom inside the housing. The fluid container 600 completes one longitudinal reciprocating motion in the inhaler 500 during a process of using the nebulising assembly 400 to complete one nebulization inhalation, and the drive part 501 is configured to drive or actuate the dose indication device when the fluid container 600 reciprocates. The structures of the inhaler 500 and the nebulising assembly 400 are the prior art, and details are not described herein again.

In this embodiment, the drive part 501 is a cylindrical small rod extending upward from the bottom of the housing. It can be understood that, in another embodiment of the present application, the drive part 501 may be fixedly connected or detachably connected to the bottom of the housing in a direct or indirect manner (for example, by using another connecting component), and the shape of the drive part 501 may alternatively be another shape, e.g., a prismatic small rod.

At present, in the conventional inhaler 500, the fluid container 600 moves along the central axis of the housing when the nebulising assembly 400 is used, such that the drive part 501 at the bottom of the housing moves relative to the fluid container 600. During the relative movement, the drive part 501 actuates the dose indication device at the bottom of the fluid container 600, and the dose indication device displays the number of uses or the remaining number of uses (hereinafter referred to as count values). However, in actual production, it is inevitable that the dimensions of the components such as the drive part 501 and the dose indication device may have some deviations. For example, for different batches of products, the length of the cylindrical rod as the drive part 501 and the height of an outer shell 100 of the dose indication device may not be exactly the same, which leads to a deviation in the initial distance and/or a deviation in the relative movement distance between the drive part 501 and the fluid container 600 (the fluid container is connected with the dose indication device, and more specifically, the bottom thereof is connected with the dose indication device) after the assembly of different batches of devices and different batches of fluid containers 600, resulting in inaccurate display of the dose indication device and a low fault tolerance rate of the device. For another example, for different products, fluid containers 600 with different specifications are likely to be selected, and the heights of the fluid containers 600 are likely to be different. For example, when the same batch of inhalers 500 are used to assemble fluid containers 600 with different specifications, there is a large difference in the initial distance and/or a large difference in the relative movement distance between the drive part 501 and the fluid container 600 (the bottom of which is connected with the dose indication device), resulting in inaccurate or even failure of the dose indication device. In this case, it is highly likely that the parameters of the components in the dose indication device need to be readjusted, resulting in poor versatility of the device.

The present application provides a dose indication device for an inhaler 500, as shown in FIGs. 2-3, which includes an outer shell 100 and a base 200 connected to each other, and an empty cavity located between the outer shell 100 and the base 200. In order to accommodate most inhalers 500 on the market, the outer shell 100 is preferably cylindrical.

The dose indication device is connected to a fluid container 600 through the outer shell 100. Exemplarily, a fluid container connecting part 110 is arranged at the end of the outer shell 100 away from the base 200, and the fluid container connecting part 110 is arranged coaxially (central axis) with the housing of the inhaler 500 for connecting the fluid container 600. In an embodiment of the present application, the connection manner is a snap-fit connection. It can be understood that another connection manner, such as a direct connection or an indirect connection, or a fixed connection or a detachable connection, can achieve the objectives of the present application.

In another embodiment of the present application, the fluid container connecting part 110 is fixedly connected to the fluid container 600, and when the fluid container 600 needs to be replaced with a new one, the fluid container 600 and the dose indication device are replaced together.

In this embodiment, the dose indication device is connected to the bottom of the fluid container 600 through the fluid container connecting part 110, and the dose indication device may reciprocate longitudinally (move up and down) along with the fluid container 600.

A push rod accommodating part 210 protruding toward the outer shell 100 is arranged at the center of the base 200, a push rod hole 211 penetrating the base 200 and an end surface 201 of the push rod accommodating part 210 away from the base 200 is provided at the center of the end surface 201, the push rod 220 movably passes through the push rod hole 211, and an elastic assembly 221 (including, but not limited to, a compression spring, etc.) is further arranged between the push rod 220 and the end surface 201. Specifically, the push rod accommodating part 210 has an accommodating space for accommodating the push rod 220, a stop part 222 is arranged on the push rod 220 (in a direction away from the end surface 201), one end of the elastic assembly 221 abuts against the stop part 222, and the other end thereof abuts against the end surface 201. The present application does not limit the shape of the stop part 222, which may be annular or other shapes, as long as it can limit the elastic assembly 221. Preferably, the stop part 222 is located at the bottom of the push rod 220. A direction extending from the base 200 to the fluid container connecting part 110 (a direction in which the central axis of the inhaler 500 is located, or a direction in which the central axis of the dose indication device is located) is defined as an up-down direction (a vertical direction, a perpendicular direction, or a longitudinal direction), a direction orthogonal to the up-down direction is defined as a horizontal direction (a lateral direction, such as a left-right direction in FIGs. 2 and 3); and a direction indicated by an arrow A in FIGs. 2 and 3 is defined as an upper direction.

In this embodiment, the drive part 501 is coaxially arranged with the push rod hole 211, and when the dose indication device moves downward, the drive part 501 abuts against and pushes the push rod 220 to move upward in the push rod hole 211 relative to the fluid container 600. Preferably, the length of the push rod 220 does not exceed the length of the push rod hole 211. Preferably, the push rod 220 does not protrude from the surface of the base 200 away from the outer shell 100.

A push-pull rod 310 is fixedly connected to the end of the push rod 220 passing through the push rod hole 211 and facing the outer shell 100. The push-pull rod 310 includes a push-pull rod main body 311, and a pushing rod 312 and a pulling rod 313 which are located on two sides of the push-pull rod main body 311 and are arranged opposite each other. Specifically, the push-pull rod main body 311 is fixedly connected to the push rod 220. The height of the pushing rod 312 (a length in a direction of a central axis of the housing (i.e., a vertical direction)) is less than the height of the pulling rod 313. In this embodiment, the pushing rod 312 and the pulling rod 313 are arranged opposite each other. Compared with other designs (such as the arrangement on the same side), the height and shape of the pushing rod 312 and the pulling rod 313 have better design flexibility, which is beneficial to improving the fault tolerance rate.

The pushing rod 312 includes a pushing part 314, the pushing part 314 being fixedly connected to the push-pull rod main body 311 by a first vertical rod arranged vertically; the pulling rod 313 includes a pulling part 315, the pulling part 315 being fixedly connected to the push-pull rod main body 311 by a second vertical rod arranged vertically. It can be seen from the above description that, in the vertical direction, the height of the pushing part 314 is different from the height of the pulling part 315, and the pushing part 314 is lower than the pulling part 315. In other words, the distance between the pushing part 314 and the push-pull rod main body 311 is less than the distance between the pulling part 315 and the push-pull rod main body 311. When the push rod 220 moves (moves up and down) in the push rod accommodating part 210, the push-pull rod 310 is driven to move (move up and down). In this embodiment, the pushing part 314 is fixedly connected to the push-pull rod main body 311 through two first vertical rods, and the two first vertical rods are connected to two ends of the pushing part 314, respectively; the pulling part 315 is fixedly connected to the push-pull rod main body 311 through two second vertical rods, and the two second vertical rods are connected to two ends of the pulling part 315, respectively. It can be understood that the first vertical rod and the second vertical rod may have other numbers as long as the rotational movement of the first transmission wheel 321 in the perpendicular direction is not affected.

The dose indication device further includes a transmission wheel assembly 320. The transmission wheel assembly 320 includes a first transmission wheel 321, a second transmission wheel 322, and a horizontally arranged transmission shaft 323; the first transmission wheel 321 is fixed in the middle of the transmission shaft 323 and located between the pushing rod 312 and the pulling rod 313, and the second transmission wheel 322 is fixed at an end portion of the transmission shaft 323. In order to support the transmission wheel assembly 320, the dose indication device further includes a transmission wheel assembly bracket 230, the transmission wheel assembly bracket 230 being fixed to the base 200. The transmission wheel assembly 320 is rotatably arranged in the transmission wheel assembly bracket 230. Specifically, the transmission wheel assembly bracket 230 is arranged on the outer side of the push rod accommodating part 210 and is provided with a transmission shaft groove 231 for accommodating the transmission shaft 323. The above design allows the transmission shaft 323 to rotate only on the transmission wheel assembly bracket 230 and cannot move in other directions, and moreover, the transmission shaft 323 is parallel to the pushing part 314 and the pulling part 315. A specific structure and position of the transmission wheel assembly bracket 230 are not limited in the present application, and the transmission wheel assembly 320 may alternatively be fastened in another manner.

In order to ensure that the push-pull rod 310 can actuate the transmission wheel assembly 320 and has a high fault tolerance rate, a distance D between the pushing rod 312 and the pulling rod 313 is less than an outer diameter of the first transmission wheel 321. Specifically, a ratio of the distance D to a radius R of the first transmission wheel 321 ranges from 1.3 to 1.9; in some preferred embodiments, the ratio ranges from 1.4 to 1.6; and in some more preferred embodiments, the ratio ranges from 1.4 to 1.5.

The cooperation between the push-pull rod 310 and the transmission wheel assembly 320 during operation is as follows: when the push-pull rod 310 moves upward (toward the direction close to the fluid container connecting part 110) (in a ready-to-dose state, that is, the fluid container 600 moves downward relative to the drive part 501, causing the drive part 501 to push the push rod 220 to move upward relative to the fluid container 600, thereby causing the push-pull rod 310 to move upward), the pushing part 314 of the pushing rod 312 located below moves upward to push the first transmission wheel 321 to rotate (exemplarily, to rotate counterclockwise as shown in FIG. 5). In this case, the pulling rod 313 does not block the rotation of the first transmission wheel 321, and does not apply a force to the first transmission wheel 321, or applies a slight force that does not affect the rotation of the first transmission wheel 321. When the push-pull rod 310 moves downward (in a direction away from the fluid container connecting part 110) (a priming dose state, that is, under the action of a drive spring (not shown), the fluid container 600 moves upward relative to the drive part 501, the push rod 220 moves away from the drive part 501, and the push rod 220 moves downward relative to the fluid container 600 under the action of the elastic assembly 221, thereby causing the push-pull rod 310 to move downward), the pulling part 315 of the pulling rod 313 located above moves downward to pull the first transmission wheel 321 to rotate (exemplarily, as shown in FIG. 6, to rotate counterclockwise). In this case, the pushing rod 312 does not block the rotation of the first transmission wheel 321, and does not apply a force to the first transmission wheel 321, or applies a slight force that does not affect the rotation of the first transmission wheel 321. This process is repeated, such that rotation of the first transmission wheel 321 can be implemented, and up and down movement of the push rod 220 is converted into rotation of the first transmission wheel 321. In other words, the first transmission wheel 321 is configured to be actuated to rotate in the same direction when the push-pull rod 310 moves up and down (for example, when counting is required) (that is, to rotate in one direction under the action force of the pushing part 314 and the pulling part 315, which is a counterclockwise direction in the figure).

In order to enable the push-pull rod 310 to push and pull the first transmission wheel 321 more smoothly and improve the fault tolerance rate, a ratio of a height difference ΔH of the push-pull rod 310 (i.e., the height difference ΔH between the pushing rod 312 and the pulling rod 313) to the radius R of the first transmission wheel 321 is greater than or equal to 0.4, or greater than or equal to 0.5, or greater than or equal to 0.6, or greater than or equal to 0.7, or greater than or equal to 0.8, or greater than or equal to 0.9, or greater than or equal to 1. In some preferred embodiments, the ratio ranges from 0.6 to 0.8. A ratio of the height H of the pushing rod 312 (i.e., the distance H between the top end of the pushing part 314 and the push-pull rod main body 311) to the radius R of the first transmission wheel 321 ranges from 0.6 to 1.3; in some preferred embodiments, the ratio ranges from 0.8 to 1.2; and in some more preferred embodiments, the ratio ranges from 1.0 to 1.1.

In order to prevent reverse rotation of the first transmission wheel 321, the dose indication device is further provided with a first anti-reverse-rotation mechanism, which includes, but is not limited to, lock pawls 101. The present application takes the lock pawls 101 as an example for illustration. Exemplarily, the lock pawls 101 are arranged in the empty cavity (exemplarily, specifically on the top surface of the outer shell 100 (i.e., a surface provided with the fluid container connecting part 110)) and extend towards the first transmission wheel 321. In this example, the number of the lock pawls 101 is two, but the present application is not limited thereto. Those skilled in the art can adjust the number, position and structure of the lock pawls 101 on the basis of actual conditions. Preferably, the dose indication device includes two lock pawls 101 extending towards two sides of a tooth of the first transmission wheel 321, respectively.

In this embodiment, the lock pawls 101 are each of an elastic sheet structure, and have a minimum thickness at a position closest to the tooth of the first transmission wheel 321 (specifically, as shown in FIGs. 5-6, the thickness decreases on the side close to the pushing rod 312). When the first transmission wheel 321 rotates in the same direction, the lock pawls 101 are slightly elastically deformed and provide an audible feedback to prompt the user that aerosol inhalation is being performed. When the first transmission wheel 321 rotates in a reverse direction, the lock pawls 101 are greatly deformed and generate a large reaction force, thereby preventing reverse rotation of the first transmission wheel 321.

In some embodiments of the present application, the present application does not limit the specific shape of the first transmission wheel 321, as long as the push-pull rod 310 can actuate the first transmission wheel 321, that is, as long as another tooth can smoothly rotate below the pulling rod 313 in the rotation process in which the pushing rod 312 pushes one tooth upward, and another tooth can smoothly rotate above the pushing rod 312 in the rotation process in which the pulling rod 313 pushes one tooth downward.

In some other preferred embodiments of the present application, the teeth of the first transmission wheel 321 are deflected towards the pushing rod 312. As shown in FIG. 8, in the cross-sectional view, an included angle between a connection line between the center of the bottom portion (a portion close to the center of the first transmission wheel 321) of the tooth and the center of the first transmission wheel 321 and a connection line between the center (in the figure, the top portion of the tooth is a point) of the top portion (a portion away from the rotation axis of the first transmission wheel 321) of the tooth and the center of the first transmission wheel 321 is a deflection angle α, and the deflection angle α is 0° to 70°; in some preferred embodiments, the deflection angle α is 15° to 65°; in some further preferred embodiments, the deflection angle α is 30° to 65°; and in some more preferred embodiments, the deflection angle α is 45° to 55°. Preferably, the (cross-sectional) edge of the tooth is an arc. The advantages of such a design are that, in one aspect, the pushing part 314 and the pulling part 315 provide a more stable acting force on the teeth, thereby improving the stability of the entire device structure; in another aspect, the shapes of the pushing part 314 and the pulling part 315 do not need to mesh with the shapes of the teeth, thereby improving the flexibility of the design, and providing a high fault tolerance rate in both shape and size, which is especially suitable for industrial production and reduces the production cost; and in yet another aspect, the anti-reverse-rotation effect of the lock pawls 101 on the first transmission wheel 321 is facilitated, as shown in FIGs. 5-6, when the first transmission wheel 321 rotates in the same direction, when the teeth of the first transmission wheel 321 are in contact with the lock pawls 101, the deflection design of the teeth is more conducive to slight elastic deformation of the lock pawls 101 towards the pulling rod 313, thereby facilitating further co-rotation of the first transmission wheel 321; when the first transmission wheel 321 rotates in the reverse direction, when the teeth of the first transmission wheel 321 are in contact with the lock pawls 101, the deflection design of the teeth is more unfavorable to deformation of the lock pawls 101 towards the pushing rod 312, thereby facilitating generation of a relatively large reaction force by the lock pawls 101 and preventing reverse rotation of the first transmission wheel 321.

In some embodiments of the present application, the pushing part 314 and the pulling part 315 are arranged horizontally. In some other embodiments of the present application, in order to further provide a more stable acting force on the teeth, the pushing part 314 and the pulling part 315 are both arranged obliquely in a horizontal direction towards the pushing rod 312. As shown in FIG. 6, in the cross-sectional view, an included angle between a surface of the pushing part 314 and/or the pulling part 315 in contact with the teeth of the first transmission wheel 321 and a horizontal line (shown by a dotted line in the figure) is an inclination angle β, and the inclination angle β is 0° to 60°; in some preferred embodiments, the inclination angle β is 0° to 45°; in some further preferred embodiments, the inclination angle β is 10° to 30°; and in some more preferred embodiments, the inclination angle β is 15° to 25°.

The number of the teeth of the first transmission wheel 321 is not limited in the present application, and those skilled in the art may make a design selection on the basis of an actual situation. The rotation angle of the first transmission wheel 321 may be changed by changing the number of the teeth of the first transmission wheel 321. Preferably, the number of the teeth is 3 to 6.

In order to show the count values (the number of uses or the remaining number of uses) of the fluid container 600 in the inhaler 500, the dose indication device further includes a dose indicator ring 330 coaxially arranged with the base 200 and rotatable around the central axis of the base 200. Exemplarily, the dose indicator ring 330 is annular. The bottom of the dose indicator ring 330 abuts against the base 200, and the top thereof is provided with gear teeth 331 engaged with the second transmission wheel 322. In order to limit the position of the dose indicator ring 330, the base 200 is provided with a plurality of limiting sheets 240, and the plurality of limiting sheets 240 are arranged on the outer periphery of the dose indicator ring 330 to avoid misalignment of the dose indicator ring 330. The outer periphery of the dose indicator ring 330 is further provided with graduations for displaying count values, and by controlling the rotation direction of the dose indicator ring 330 and the order of the graduations, it is possible to display the number of times the fluid container 600 in the inhaler 500 has been used or the remaining number of uses.

In order to facilitate user's observation of the count values, an observation window 120 is arranged on a side surface of the outer shell 100, and the position of the observation window 120 corresponds to the position of the dose indicator ring 330.

When the push rod 220 moves up and down, the push-pull rod 310 is driven to move up and down, the push-pull rod 310 actuates the first transmission wheel 321, the first transmission wheel 321 drives the transmission shaft 323 to rotate, and the transmission shaft 323 drives the second transmission wheel 322 to rotate. The dose indicator ring 330 rotates by meshing transmission of the second transmission wheel 322, thereby realizing dose indication.

In some embodiments of the present application, the present application does not limit the number of the teeth of the second transmission wheel 322 and the number of the gear teeth 331 of the dose indicator ring 330, and those skilled in the art may make a design selection on the basis of an actual situation.

In some preferred embodiments of the present application, the number of the teeth of the second transmission wheel 322 is less than or equal to the number of the teeth of the first transmission wheel 321. Preferably, the number of the teeth of the second transmission wheel 322 is less than the number of the teeth of the first transmission wheel 321. In some embodiments of the present application, the number of the teeth of the second transmission wheel 322 is 2 to 5; and in one preferred embodiment of the present application, the number of the teeth of the second transmission wheel 322 is 2. The advantages of such a design are that the transmission ratio of the device can be adjusted by using the difference in the number of the teeth of the first transmission wheel 321 and the second transmission wheel 322, which increases the flexibility of the entire device; in one aspect, the number of corresponding gear teeth 331 of the dose indicator ring 330 can be significantly reduced, such that the structural strength of the gear teeth 331 can be increased in the limited space, the structural stability of the device can be improved, and the production difficulty and production cost can be reduced; and in another aspect, the rotation angle corresponding to an individual gear tooth 331 of the dose indicator ring 330 can be increased, and the meshing space of the gear teeth 331 and the teeth of the second transmission wheel 322 can be increased, which further improves the fault tolerance rate of the device.

The actuation process of the dose indicator ring 330 will be described below in conjunction with specific examples.

In this example, there are five teeth of the first transmission wheel 321, the five teeth are evenly distributed on the outer periphery of the first transmission wheel 321, and there are two teeth of the second transmission wheel 322, with an included angle between the two teeth of 144°.

When the user uses the inhaler 500, the nebulising assembly 400 is used for atomization once, the fluid container 600 moves back and forth longitudinally once, and the push rod 220 moves up and down once under the action of the drive part 501 and the elastic assembly 221. Since the first transmission wheel 321 is provided with a total of five teeth, and the second transmission wheel 322 is provided with a total of two teeth, when the push rod 220 moves up and down twice, the second transmission wheel 322 actuates the dose indicator ring 330 to rotate once; and then, when the push rod 220 moves up and down three times, the second transmission wheel 322 actuates the dose indicator ring 330 to rotate once. The process is repeated in sequence. That is, two rotations of the dose indicator ring 330 per five nebulizations using the nebulising assembly 400 are achieved.

Obviously, those skilled in the art may change the number of the teeth of the first transmission wheel 321 and the second transmission wheel 322, and the number of the gear teeth 331 of the dose indicator ring 330 to change the number of rotations and the rotation angle of the dose indicator ring 330, which is not specifically limited in the present application.

In order to prevent reverse rotation of the dose indicator ring 330, the dose indication device further includes a second anti-reverse-rotation mechanism, which includes, but is not limited to, a ratchet mechanism. The present application takes the ratchet mechanism as an example for illustration. A ratchet mechanism is further arranged on the inner periphery (inner side) of the dose indicator ring 330 such that the dose indicator ring 330 can only rotate in the same direction (in one direction under actuation of the second transmission wheel 322) under actuation of the second transmission wheel 322, thereby preventing display errors. Specifically, the ratchet mechanism includes a ratchet 332 and check pawls 333. Exemplarily, the ratchet 332 is arranged on the inner periphery of the dose indicator ring 330, the check pawls 333 are arranged on the outer periphery of the push rod accommodating part 210, and the ratchet 332 and the check pawls 333 are provided at the same height. In some embodiments of the present application, the number of the check pawls 333 is at least two; and in some preferred embodiments of the present application, the number of the check pawls 333 is two, and the two check pawls are arranged symmetrically with respect to each other. In the present application, the counting and display accuracy of the dose indication device of the present application can be effectively ensured by adopting a double anti-reverse-rotation design, specifically, by preventing reverse rotation of the first transmission wheel 321 by using the lock pawls 101 and preventing reverse rotation of the dose indicator ring 330 by using the ratchet mechanism.

Although the above-described dose indication device may inform the user of the usage of the fluid container 600, the user may forget to observe the usage of the fluid container. When the fluid in the fluid container 600 reaches or exceeds the predetermined number of uses, especially when the fluid is nearly depleted or has been depleted, if the user continues to use the fluid container 600 without timely replacing the fluid container 600 with a new one, it is likely that the ejected dose is different from the predetermined dose, which may cause a risk of medication.

In order to avoid the occurrence of the above-mentioned problem, the dose indication device of the present application further includes a locking mechanism with a rotatable stopper 340. The locking mechanism is configured to prevent movement of the push rod 220 and/or further rotation of the dose indicator ring 330 after a predetermined number of uses of the fluid container 600. It should be noted that the locking mechanism of the present application can be applied not only to the inhaler 500 described above, but also to various inhalers 500 with the drive part 501 and/or the dose indicator ring 330 in the related art, as long as the stopper 340 of the locking mechanism can prevent movement of the drive part 501 (or the push rod 220) and/or the dose indicator ring 330. For example, when the dose indication device includes a locking mechanism, the base 200 is provided with a vertically arranged stopper rotating shaft 250, and the stopper 340 is rotatably sleeved on the stopper rotating shaft 250, such that the stopper 340 can rotate in the horizontal direction. The stopper 340 includes an abutment part 341, a limiting part, and a pushing abutment part 343, the abutment part 341 and the limiting part being located on two sides of the stopper rotating shaft 250, and the pushing abutment part 343 being arranged close to the dose indicator ring 330.

A stopper opening hole (not shown in the figure) communicating with the push rod hole 211 is provided on a side surface of the push rod accommodating part 210, and the stopper opening hole is provided corresponding to the abutment part 341, such that when the stopper 340 rotates, the abutment part 341 of the stopper can pass through the stopper opening hole to abut against the push rod 220. In this case, the stopper 340 is located in an abutment position, and the abutment part 341 abuts against the push rod 220 to prevent movement of the push rod 220. In this embodiment, the abutment part 341, when in the preset position, does not pass through the stopper opening hole, and the abutment part 341, when in the abutment position, passes through the stopper opening hole and at least partially protrudes from the push rod hole 211, thereby preventing the push rod 220 from moving up and down in the push rod hole 211.

In some embodiments of the present application, in the abutment position, the bottom of the push rod 220 is located below the stopper opening hole, and the abutment part 341 passes through the stopper opening hole to prevent the push rod 220 from moving downward; in some other embodiments of the present application, in the abutment position, the bottom of the push rod 220 is located above the stopper opening hole, and the abutment part 341 passes through the stopper opening hole to prevent the push rod 220 from moving upward; and in some other embodiments of the present application, the abutment part 341 prevents the drive part 501 from contacting the push rod 220, thereby preventing the drive part 501 from actuating the push rod 220.

In addition, the hole wall of the stopper opening hole also has a limiting effect on the rotation angle of the stopper 340, such that when the abutment part 341 abuts against the push rod 220, the stopper 340 cannot continue to rotate, and thus the pushing abutment part 343 provides a reverse blocking effect on the pushing member 334, and the dose indicator ring 330 cannot continue to rotate, thereby locking the dose indicator ring 330.

In order to retain the stopper 340 in the preset position before the dose indicator ring 330 rotates by a preset angle, the base 200 is provided with a limiting mechanism configured to retain the stopper 340 in the preset position, and the limiting mechanism is provided corresponding to the limiting part, such that the stopper 340 is located in the preset position where the abutment part 341 does not pass through the stopper opening hole, and at this time, the stopper 340 does not affect the movement of the push rod 220.

It should be noted that specific structures of the limiting mechanism and the limiting part in the present application may be implemented in a plurality of forms.

For example, in an embodiment of the present application, when the limiting part is a semicircular boss 342, the limiting mechanism may be a limiting slot 251 provided on the base 200, and shapes of the boss 342 and the limiting slot 251 are correspondingly set, such that the boss 342 is engaged in the limiting slot 251 when the stopper 340 is in the preset position.

Alternatively, the limiting mechanism is an elastic structure (such as a strip-shaped or block-shaped plastic elastic sheet) protruding from the base 200. Specifically, the limiting mechanism is an elastic sheet 252 that is arranged on the surface of the base 200 facing the stopper 340 and extending obliquely toward the stopper 340. In this case, a portion of the stopper 340 facing the elastic sheet 252 is used as the limiting part, and the stopper 340 may be temporarily fixed in the preset position by abutment of the limiting part and the elastic structure. When the stopper 340 is in the preset position, in order to prevent the abutment part 341 from moving to a position away from the stopper opening hole, the base 200 is further provided with a protruding point 253 for preventing the abutment part 341 from moving to a position away from the stopper opening hole, and the protruding point 253 abuts against the abutment part 341 when the stopper 340 is in the preset position.

In order to push the pushing abutment part 343 to rotate the stopper 340 from the preset position to the abutment position, the locking mechanism includes a pushing member 334 arranged on the inner periphery of the dose indicator ring 330; the pushing member 334 is configured to push the stopper 340 from the preset position to the abutment position after the dose indicator ring 330 rotates by a preset angle; the stopper 340, when in the preset position, does not prevent the push rod 220 from moving, and the stopper 340, when in the abutment position, prevents the push rod 220 from moving.

In this embodiment, before the dose indicator ring 330 rotates by a preset angle, the stopper 340 is in the preset position, and the pushing member 334 does not interact with the stopper 340. Only after the dose indicator ring 330 rotates by a preset angle, the pushing member 334 comes into contact with the stopper 340 to push the stopper 340 from the preset position to the abutment position.

In a preferred embodiment of the present application, the pushing member 334 is a protrusion protruding from the inner periphery of the dose indicator ring 330 towards the stopper 340, the protrusion is arranged corresponding to the pushing abutment part 343, and the protrusion is configured to push the pushing abutment part 343 after the dose indicator ring 330 rotates by a preset angle. By rotating the dose indicator ring 330, the protrusion may abut against the pushing part 343, and the continued rotation of the dose indicator ring 330 pushes the stopper 340 to rotate, such that the abutment part 341 passes through the stopper opening hole and abuts against the push rod 220 to prevent the push rod 220 from moving. Similarly, at the same time, the pushing abutment part 343 provides a reverse blocking effect on the pushing member 334, such that the dose indicator ring 330 cannot continue to rotate, the locking of the dose indicator ring 330 is realized, and finally the fluid container 600 is effectively and firmly locked, thereby preventing the user from misoperation.

The preset angle may be adjusted by adjusting the initial position of the protrusion, to adapt to different use cases.

It should be noted that the height of the pushing member 334 is different from that of the ratchet mechanism, so the pushing member and the ratchet mechanism will not affect each other.

In an embodiment of the present application, the dose indication device further includes a stopping member. When in the abutment position, the stopping member prevents further rotation of the stopper 340, and at the same time, the pushing abutment part 343 prevents further rotation of the pushing member 334, thereby locking the dose indicator ring 330. In a preferred embodiment of the present application, the stopping member is a convex point arranged on the base 200. The stopper 340, when in the abutment position, abuts against the convex point. In a preferred embodiment of the present application, as described above, a hole wall on one side of the stopper opening hole may serve as a stopping member. In a preferred embodiment of the present application, the limiting slot 251 may also serve as a stopping member, and the stopper 340, when in the abutment position, abuts against a portion of the limiting slot 251.

The locking mechanism in the dose indication device of the present application provides a double locking effect, in one aspect, the locking mechanism locks the count of the dose indication device by the stopper 340 preventing the up and down movement of the push rod 220, and in another aspect, the locking mechanism prevents further rotation of the stopper 340 by the stopping member, such that the pushing abutment part 343 provides a reverse blocking effect on the pushing member 334, thereby preventing the rotation of the dose indicator ring 330 and thus locking the indication of the dose indication device. Finally, when a medicinal solution in the fluid container 600 reaches or exceeds the predetermined number of uses, the fluid container 600 is effectively and firmly locked, prompting the user to replace the fluid container 600 with a new one, thus ensuring the safety of medication for the user.

The foregoing shows and describes the basic principles, main features, and advantages of the present application. It should be understood by those skilled in the art that the present application is not limited to the embodiments described above. The embodiments described above and the descriptions in the specification are merely illustrative of the principles of the present application. Various changes and improvements may be made to the present application without departing from the spirit and scope of the present application, and these changes and improvements all fall within the scope of protection of the present application.

## Claims

1. A dose indication device for an inhaler, the inhaler comprising:
a housing for receiving a fluid container and a nebulising assembly for nebulising a fluid, a drive part for actuating a dose indication device being arranged at a bottom inside the housing;
wherein the dose indication device comprises an outer shell and a base connected to each other, and an empty cavity located between the outer shell and the base; the dose indication device is connected to the fluid container through the outer shell, a push rod accommodating part protruding toward the outer shell is arranged at a center of the base, a push rod hole penetrating the base and an end surface of the push rod accommodating part away from the base is provided at a center of the end surface, a push rod movably passes through the push rod hole, and a push-pull rod is fixedly connected to an end of the push rod passing through the push rod hole and facing the outer shell; the push-pull rod comprises a push-pull rod main body, and a pushing rod and a pulling rod which are located on two sides of the push-pull rod main body and arranged opposite each other, a height of the pushing rod being lower than a height of the pulling rod;
the dose indication device further comprises a transmission wheel assembly, and the transmission wheel assembly comprises a first transmission wheel, a second transmission wheel, and a horizontally arranged transmission shaft; the first transmission wheel is fixed in a middle of the transmission shaft and located between the pushing rod and the pulling rod, a distance between the pushing rod and the pulling rod is smaller than an outer diameter of the first transmission wheel, and the first transmission wheel is configured to be actuated by the pushing rod or the pulling rod to rotate in a same direction when the push-pull rod moves up and down; the second transmission wheel is fixed at an end portion of the transmission shaft;
the dose indication device further comprises a dose indicator ring coaxially arranged with the base and rotatable around a central axis of the base, a bottom of the dose indicator ring abutting against the base, gear teeth engaged with the second transmission wheel being arranged at a top of the dose indicator ring, and graduations for displaying count values being arranged on an outer periphery of the dose indicator ring.

2. The dose indication device according to claim 1, wherein a stop part is arranged at a bottom of the push rod, an elastic assembly is further arranged between the push rod and the end surface, one end of the elastic assembly abuts against the stop part, and the other end thereof abuts against the end surface.

3. The dose indication device according to claim 1 or 2, wherein the pushing rod comprises a pushing part, the pushing part being fixedly connected to the push-pull rod main body by a first vertical rod arranged vertically; the pulling rod comprises a pulling part, the pulling part being fixedly connected to the push-pull rod main body by a second vertical rod arranged vertically; a height of the pushing part is lower than a height of the pulling part.

4. The dose indication device according to any one of claims 1 to 3, wherein a ratio of the distance between the pushing rod and the pulling rod to a radius of the first transmission wheel ranges from 1.3 to 1.9; and/or
a ratio of a height difference between the pushing rod and the pulling rod to a radius of the first transmission wheel ranges from 0.6 to 0.8; and/or
a ratio of the height of the pushing rod to a radius of the first transmission wheel ranges from 0.6 to 1.3.

5. The dose indication device according to any one of claims 1 to 4, wherein teeth of the first transmission wheel are deflected towards the pushing rod, with a deflection angle α of 0° to 70°.

6. The dose indication device according to any one of claims 3 to 5, wherein the pushing part and the pulling part are arranged horizontally, or the pushing part and the pulling part are arranged obliquely in a horizontal direction towards the pushing rod, with an inclination angle β of 0° to 60°.

7. The dose indication device according to any one of claims 1 to 6, wherein the dose indication device further comprises a transmission wheel assembly bracket fixed to the base, the transmission wheel assembly being rotatably arranged in the transmission wheel assembly bracket.

8. The dose indication device according to any one of claims 1 to 7, wherein a number of the teeth of the first transmission wheel is 3 to 6, and a number of teeth of the second transmission wheel is less than the number of the teeth of the first transmission wheel.

9. The dose indication device according to any one of claims 1 to 8, wherein the dose indication device further comprises a first anti-reverse-rotation mechanism, the first anti-reverse-rotation mechanism being arranged in the empty cavity and extending towards the first transmission wheel to prevent reverse rotation of the first transmission wheel.

10. The dose indication device according to claim 9, wherein the dose indication device further comprises a second anti-reverse-rotation mechanism, the second anti-reverse-rotation mechanism being arranged on an inner side of the dose indicator ring to prevent reverse rotation of the dose indicator ring.

11. The dose indication device according to claim 9 or 10, wherein the first anti-reverse-rotation mechanism comprises at least one lock pawl, the lock pawls being arranged on a top surface of the outer shell.

12. The dose indication device according to claim 10, wherein the second anti-reverse-rotation mechanism is a ratchet mechanism; a ratchet is arranged on an inner periphery of the dose indicator ring, and mating check pawls are arranged in the empty cavity.

13. The dose indication device according to any one of claims 1 to 12, wherein the dose indication device further comprises a locking mechanism, the locking mechanism being configured to prevent movement of the push rod after the fluid container has been used a predetermined number of times.

14. The dose indication device according to claim 13, wherein the locking mechanism comprises a rotatable stopper, a vertically arranged stopper rotating shaft is arranged on the base, the stopper is rotatably sleeved on the stopper rotating shaft, and the stopper comprises an abutment part, a limiting part, and a pushing abutment part, the abutment part and the limiting part being located on two sides of the stopper rotating shaft, and the pushing abutment part being arranged close to the dose indicator ring.

15. The dose indication device according to claim 14, wherein the locking mechanism comprises a pushing member arranged on an inner periphery of the dose indicator ring, the pushing member is configured to push the stopper from a preset position to an abutment position after the dose indicator ring rotates by a preset angle, the stopper, when in the preset position, does not prevent the movement of the push rod, and the stopper, when in the abutment position, prevents the movement of the push rod.

16. The dose indication device according to claim 15, wherein the pushing member is a protrusion protruding from the inner periphery of the dose indicator ring towards the stopper, the protrusion is arranged corresponding to the pushing abutment part, and the protrusion is configured to push the pushing abutment part after the dose indicator ring rotates by a preset angle.

17. The dose indication device according to claim 15 or 16, wherein a stopper opening hole communicating with the push rod hole is provided on a side surface of the push rod accommodating part, the stopper opening hole is provided corresponding to the abutment part such that the abutment part can pass through the stopper opening hole when the stopper rotates, the abutment part, when in the preset position, does not pass through the stopper opening hole, and the abutment part, when in the abutment position, passes through the stopper opening hole and at least partially protrudes from the push rod hole.

18. The dose indication device according to any one of claims 15 to 17, wherein a limiting mechanism is arranged on the base, the limiting mechanism is arranged corresponding to the limiting part, and the limiting mechanism is configured to retain the stopper in the preset position.

19. The dose indication device according to claim 18, wherein the limiting part is a semicircular boss, the limiting mechanism is a limiting slot provided on the base, and the boss and the limiting slot are correspondingly provided in a matching shape.

20. The dose indication device according to claim 18, wherein the limiting mechanism is an elastic sheet arranged on a surface of the base facing the stopper and extending obliquely toward the stopper, the base is provided with a protruding point for preventing the abutment part from moving away from the stopper opening hole, and the protruding point, when in the preset position, abuts against the abutment part.

21. The dose indication device according to any one of claims 15 to 20, wherein the dose indication device further comprises a stopping member, and the stopping member, when in the abutment position, prevents further rotation of the stopper.

22. A fluid container, a bottom of the fluid container being connected to the dose indication device according to any one of claims 1 to 21.

23. An inhaler, wherein the housing of the inhaler being internally provided with the dose indication device according to any one of claims 1 to 21.
